# FASCICULE DE BREVET EUROPEEN

(11) **EP 0 644 773 B1**
(45) Date de publication et mention de la délivrance du brevet: **26.06.2002**
(21) Numéro de dépôt: 94907595.6
(22) Date de dépôt: 18.02.1994
(51) Int. Cl.: A61K 35/74, A61K 31/70, A61P 37/02

(54) **COMPLEXE IMMUNOMODULATEUR ANTI-SIDA**
Anti-Aids Immunomodulatorkomplex
ANTI-AIDS IMMUNOMODULATOR COMPLEX

(30) Priorité: 31.03.1993 FR 9303879
(43) Date de publication de la demande: 29.03.1995
(73) Titulaire: TOROSSIAN, Fernand Narbey, F-31400 Toulouse (FR)
(72) Inventeur: TOROSSIAN, Fernand Narbey, F-31400 Toulouse (FR)
(74) Mandataire: Morelle, Guy Georges Alain
(86) Numéro de dépôt international: FR9400184
(87) Numéro de publication internationale: WO9422462

(56) Documents cités:
- EP-A- 0 013 851
- EP-A- 0 031 285
- FR-A- 2 388 563

## Description

La présente invention concerne un complexe vaccinal thérapeutique antisidaïque, qui possède un pouvoir vaccinant lié à la présence d'antigènes non spécifiques.

Il est bien connu, en bactériologie, que les antigènes de surface des parois, des membranes ou des capsules (combinés ou libérés sous forme soluble dans le milieu de culture) sont de nature glycoprotéique, polypeptidique ou polysaccharidique.

Des vaccins récents ont été largement publiés, associant à l'ARN (d'origine ribosomale) de telles substances membranaires protéoglycaniques ou polysaccharidique, extraites de germes pathogènes.

Ces vaccins utilisent des antigènes spécifiques correspondant à des affections microbiennes spécifiquement déterminées.

Or, le pouvoir antigénique est essentiellement lié au niveau de l'ARN (des ribosomes en particulier) des cellules microbiennes, entre autres. Les CIC (Cellules Immunologiquement Compétentes) utilisent directement ces ARN comme transporteurs actifs.

Pour bâtir notre nouveau complexe vaccinal, au lieu d'utiliser un antigène sérotype bactérien, nous avons couplé (grâce à des liaisons de préférence covalentes) l'ARN (d'origine ribosomale de préférence) à une séquence d'acides aminés de nature glycoprotidique, présente dans le collagène de type III (chez l'homme, le collagène représente approximativement le tiers des protéines de l'organisme; le type III a été choisi pour sa séquence d'acides aminés et parce qu'il est présent essentiellement dans le derme et la paroi vasculaire).

Par ailleurs, l'ARN est déjà utilisé dans la préparation de vaccins acellulaires (cf. Infect and Immunity, 1, 574-82, 1970). Cet ARN est stabilisé par des facteurs associatifs.

Dans notre complexe, nous utilisons comme stabilisant des fractions membranaires cellulaires issues des mêmes germes que ceux qui ont servi à l'élaboration de l'ARN ribosomal. Ces fractions membranaires contiennent la totalité des substances peptidoglycaniques et sont connues en outre comme adjuvants d'immunité.

Il n'est, contrairement aux habitudes, pas nécessaire d'avoir les mêmes fractions membranaires (glucopolysacchariques ou protéoglycanes) issues des mêmes germes microbiens que ceux qui ont servi à fournir l'ARN par extraction de leurs ribosomes.

### Constituants du complexe vaccinal objet de l'invention

### A - Les ARN d'origine ribosomale utilisables peuvent être extraits des souches suivantes, cette liste n'étant pas limitative :

- Klebsiella pneumoniae
- Streptococcus (pneumoniae et pyogènes)
- Staphilococcus aureus
- Serratia marcescens
- Escherichia coli
- Salmonella typhimurium
- Corynebacterium (granulosum, parvum, acnes)
- Mycobacterium (tuberculosis, smegmatis, chelonei)
- Hemophilus influenzae
- Pneumocoque type II
- Rothia dento cariosus
- Bacterium coli
- Shigella dysentariae
- Enterococcus
- Nocardia (astéroïdes, brasiliensis, rhodocrans, opaca, rubra)
- Bacille de Calmette et Guerin

Les poids moléculaires moyens de ces ARN se situent entre 5.104 et 108 Dalton.

De multiples procédés industriels existent pour la préparation de l'ARN ; nous citerons comme exemple le procédé d'extraction d'ARN décrit dans Infect. and Immunity, 1, 574-82, 1970 : les bactéries sont broyées puis soumises à une précipitation fractionnée, les protéines ribosomales sont solubilisées, l'ARN précipité est traité par Pronase et, enfin, purifié par chromatographie échangeuse d'ions.

Si l'ARN est obtenu par voie enzymatique, la purification finale peut être faite par chromatographie de tamisage moléculaire. Voir notamment à ce sujet :
- C. EHRESMAN (1972) - Biochimie, 54, 901
- H. KAGAWA (1972) - J. Biochem., (1972), 827
- M. SANTER (1973) - J. Bact., 116, 1304
- NOMURA (1974) - Ribosomes - Ed. Cold Spring Harbor Laboratory.

### B - Les fractions membranaires de cellules bactériennes utilisables peuvent être extraites des souches suivantes, les listes données n'étant pas limitatives :

### 1 - pour les polysaccharides capsulaires

- Klebsiella pneumoniae
- Streptococcus pneumoniae
- Hemophilus influenzae
- Escherichia coli

### Klebsiella pneumoniae

- C. ERBING, L. KENNE, B. LINBERG, J. LONNGREN (1976) - Structural studies of the capsular polysaccharide of Klebsiella pneumoniae type I (Carbohydr. Res., 50 (1976) 115-20).
- W. NIMMICH (1968) - Zur Isolierung und qualitativen Bausteianalyse der K. Angigen von Klebsiellen (Med. Mikrobio. und Immunol., 154, 117, 131).
- C. RICHARD (1973) - Etude antigénique et biochimique de 500 souches de Klebsiella (Ann. Biol. Clin., 1973).

### Streptococcus pneumoniae

- F. KAUFFMANN et E. LUND (1954) (Int. Bull. Bact. Nomencl. 4, 125-28).
- FELTON et OTTINGER (J. of Bacteriology, 1942, 43, 94, 105)
- M. COLIN, M.D. MAC LEOD et coll. - Prevention of pneumococcal pneumoniae by immunization with specific capsular polysaccharides (J. Exp. Med., 1945, 82, 445-65).
- A.R. DOCHEZ et O.T. AVERY - The elaboration of specific soluble substance by Pneumococcus during growth (1971) ( J. Exp. Med. 26, 477-93).
- WEST PHAL et LUDERITZ (1952) (Z. Naturf. 7B, 148).
- C.P.J. GLAUDEMANS et H.P. TREFFERS - An improved preparation of the capsular polysaccharide from from Diplococcus pneumoniae (Carbohydr. Res. 1967, 4, 181-84).

### Hemophilus influenzae (polysaccharide capsulaire de type poly-ribosephosphate)

- P. ANDERSON et coll. (1972) - Immunization of humans with polyribosephosphate, the capsular antigen of Hemophilus influenzae type B (J. of Clin. Invest., vol. 51, 1972, 39-44).
- P. ANDERSON et coll. (1977) - Isolation of the capsular polysaccharide from supernatant of Hemophilus influenzae type B (Infect. and Immun., 1977, 15 (2), 472-77).

### Escherichia coli (polysaccharides capsulaires)

- LUDERITZ et coll. (1977) - Somatic and capsular antigens of gram-negative bacteria (Compr. Biochem. 26 A, 105-228).

### 2 - pour les lipopolysaccharides membranaires (LPS)-

Corynebacterium (avidum, bovis, diphteriae, enzymicum, equi, fascians, flaccum, faciens, flavidum, fusiforme, granulosum, helvolum, hypertrophicans, insidiosum, liquefaciens, parvum, paurometabolum, pyogènes, tumescens, xerosis)
- et les gram-moins:
- Klebsiella (pneumoniae et rhinoscleromatis)
- Salmonella typhimurium
- Serratia (marcescens, corralina, indica, plymuthica, kiluea)
- Neisseria meningitidis
- Escherichia coli
- C. ERBIN et coll. (1977) - Structural studies on the Klebsiella LPS (Carbohydr. Res., 56, 377-81).
- C.B. CASTOR et coll. (1971) - Characteristics of a highly purified pyrogenic LPS of Klebsiella pneumoniae (J. of Pharm. Sci., 60, (10), 1578-80).
- K. FUKUSHI (1964) - Extraction and purification of endotoxin from Enterobacteriaceae: a comparison of selected methods and sources (J. of Bacteriol. 87, (2), 391-400).
- G.A. LIMJUCO - Studies on the chemical composition of LPS from Neisseria meningitidis group B (J. of Gen. Microbiol. 1978, 104, 187-91).
- G.A. ADAMS (1967) - Extraction of LPS from gram-negative bacteria with DMSO (Canad. J. Biochem., 45, 422-26).
- K.G. JOHNSON (1976) - Improved techniques for the preparation of bacterial LPS (Canad. J. Microbiol. (22), 29-34).
- Y.B. KIM et coll. (1967) - Biologically active endotoxins from Salmonella mutans (J. of Bacteriol., 94, (5), 1320-26).

### 3 - pour les protéines membranaires

- Escherichia coli
- Serratia marcescens
- Streptococcus pyogènes
- Salmonella typhimurium.

### Escherichia coli

- S.F. STIRM et coll. (1967) - Episome, carried surface antigen K 88 of Escherichia coli (J. of Bacteriol., 93, (2), 731-39).
- S.J. BETZ et coll. (1977) - Chemical and biological properties of a protein rich fraction of bacterial LPS (J. of Immunol., 119, (4), 1475-81).

### Serratia marcescens

- W. WOBER (1971) - Studies on the protein moiety of endotoxin from gram-negative bacteria, characterisation of the protein-moieting isolated by acetic acid hydrolysis of endotoxin of Serratia marcescens.

### Streptococcus pyogènes

- M.K. WITTNER (1977) - Homologous and heterologous protection of mice with group-A Streptococcal M protein vaccine (Infect. and Immun., 1977, 15, (1), 104-8).

### Salmonella thyphimurium

- N. KUUSI et coll. (1979) - Immunization with major outer membrane protein in experimental salmonellosis of mice (Infect. and Immun., 1979, 25, (3), 857-62).
- C. BARBER et coll. (1972) - The protective role of proteins from Salmonella thyphimurium in infection of mice with their natural pathogen (Rev. Immunol., 36, 77-81).
- G. DELORD (1979) - Etude d'un antigène vaccinant contenu dans le surnageant de culture de Salmonella thyphimurium, souche M-206, thèse de médecine de Lyon n° 428, 1979.
- G.W. GOODMAN (1979) - Characterization of the chemical and physical properties of a novel B-lymphocyte activator endotoxin protein (Infect. and Immun., 1979, 24 (3), 685-96).

### 4 - pour les acides teichoïques et lipoteichoïques

Streptocoques, staphylocoques, et lactobacilles (la surface des bactéries gram-positives est faite d'acide teichoïque, qui est un polymère du glycérol, lié (linked) par des ponts phosphodiesters).

Les articles suivants décrivent les procédés d'obtention :
- M.M. BURGER (1966) - Teichoïc acids: antigenic determinants, chain separation, and their location in the cell wall (Microbiology 56, 910-17).
- K.W. KNOX (1973) - Immunological properties of teichoïc acids (Bacteriol. Reviews, 37, 21, 215-57).
- G.A. MILLER (1976) - Effects of streptococcal lipoteichoïc acid on host response in mice (Infect. and Immun., 1976, 13, (5), 1408-17).
- A.J. WICKEN et coll. (1975) - Lipoteichoïc acids: a new class of bacterial antigens (Science, 187, 1161-67).

### Différents dosages possibles

### A.R.N.

* FISKE et SUBBAROW - Dosage du phosphore. Chromatographie HPLC sur colonne échangeuse d'ions pour le contrôle qualitatif (J. Biol. Chem. (1926), 66, 375).

### Protéines

* LOWRY (J. Biol. Chem. (1951), 193, 265-75).

### Hexoses

* T.A. SCOTT - Dosage colorimétr. à l'anthrone (Anal. Chem. (1953), 25, 1956-61).

### Hexosamines

* L.A. ELSON (Biochem. J (1953), 27, 1824-28).

### Lipopolysaccharides

* J. JANDA et E. WORK (Febs Letters, 1971, 16 (4), 343-45).

### C - Les autres facteurs adjuvants de l'immunité, en plus des fractions membranaires, sont

- du collagène type III
- du chlorure de sodium

### Le collagène de type III utilisé est caractérisé par :

a - des séquences d'acides aminés voisines de la séquence suivante (les concentrations sont exprimées en g/kg) :

| | | |
|---|---|---|
| - acide aspartique | AA | 51,5 |
| - hydroxyproline | HP | 107,0 |
| - thréonine | TH | 16,1 |
| - sérine | SE | 27,8 |
| - acide glutamique | AG | 95,9 |
| - proline | PR | 124,0 |
| - glycine | GL | 149,0 |
| - alanine | AL | 87,9 |
| - valine | VA | 23,3 |
| - méthionine | ME | 7,5 |
| - isoleucine | IL | 14,4 |
| - leucine | LE | 27,8 |
| - tyrosine | TY | 6,7 |
| - phénylalanine | PA | 14,4 |
| - lysine | LY | 28,6 |
| - histidine | HI | 5,5 |
| - arginine | AR | 73,0 |

b - l'analyse-type suivante:

| | |
|---|---|
| - couleur | blanc jaunâtre |
| - densité apparente | 250 g/l |
| - humidité | 6 % |
| - pH d'une solution à 10 % | 6,9 |
| - viscosité Engler à 40°C (solution à 17,75 %) | 2,5 |
| - taux de matières grasses | 0,9 % |
| - taux de cendres | 2,2 % |
| - taux de Fe + Cu + Ca | 462 mg/kg |
| - métaux lourds | non décelables par spectrographie d'émission d'arc |
| - analyse élémentaire | C 46,80 % |
| | H 7,10 % |
| | N 14,96 % |

La composition du complexe vaccinal objet de l'invention, associant des ARN ou des fragments d'ARN ribosomaux, des fractions membranaires (par exemple protéoglycanes de Klebsiella pneumoniae) et du collagène de type III, complété par du chlorure de sodium et un anti-inflammatoire, permet, par administration de faibles doses n'entraînant aucune toxicité, d'obtenir un haut niveau de protection et de guérison.

La présentation préférée est la forme injectable de la composition ci-dessus présentée, mais il est possible d'utiliser d'autres présentations et/ou d'autres supports ou additifs compatibles avec une utilisation médicale.

### Mécanisme d'action du complexe vaccinal

Le complexe thérapeutique vaccinal qui vient d'être décrit présente les caractères d'une lymphokine qui, se fixant aux macrophages, inhibe la croissance intra-cellulaire du virus.

Depuis 1974-75 (A.S.et G.P. YOUMANS ), il a été constaté que l'effet d'inhibition de la réponse immune à l'ARN était réalisée par différents inhibiteurs.

Ceci nous a amené à concevoir le complexe vaccinal, objet de l'invention, qui provoque la même inhibition sur la réplication des rétrovirus V.I.H.

YOUMANS avait travaillé sur une seule souche bactérienne (Mycobacterium tuberculosis), dont le "parasitisme" est uniquement intra-cellulaire.

VENNEMAN et coll. pensent, dès 1972, que le véritable antigène pourrait être associé à l'ARN, dont le rôle serait celui d'un adjuvant. Ils vaccinent des souris avec de l'ARN ribosomal, extrait par le phénol à 65°C de ribosomes d'une souche de Salmonella typhimurium. Trente jours après cette vaccination, il s'avère que les animaux sont mieux protégés que par vaccin souche vivante (atténuée).

Il est surtout constaté que le niveau de protection est fonction de la quantité d'ARN injectée.

Il est maintenant connu que l'ARN ribosomal extrait de Streptococcus pneumoniae induit une protection de nature humorale et que l'ARN ribosomal extrait de Klebsiella pneumoniae induit une protection de nature cellulaire.

Des expériences préliminaires, faites par DUSSOURD d'HINTERLAND, FONTANGES et coll. (Division Microbiologie du Centre de Recherche du Service de Santé Militaire de Lyon, France), ont en effet démontré que ce mélange, injecté in vivo sur souris et cobayes, exerce une action sur les macrophages alvéolaires.

Cet effet "transitoire" se retrouve en dosant la phosphatase acide des plages d'hémolyse directe au contact des cellules spléniques de souris.

Les études immunologiques effectuées par DUSSOURD d'HINTERLAND à partir de vaccins ribosomaux, induisent chez la souris O.F. femelle la production d'anticorps spécifiques lorsqu'ils sont administrés à l'animal en présence d'adjuvant de FREUND (incomplet) ou de protéoglycanes membranaires de Klebsiella.

Le traitement par notre complexe vaccinal est, quant à lui, suivi d'un effet immunostimulant cellulaire et humoral, avec une action non spécifique, mais significative, sur les VLH. On peut assimiler cette action à la production d'un "V.N.F." (Virus Necrosis Factor), car c'est l'organisme du patient lui-même qui est sollicité pour rejeter les cellules infectées (l'antigénémie P24 étant, de ce fait, réduite à zéro dans la quasi-totalité des cas).

La démarche thérapeutique de S. ROSENBERG n'est pas identique à la nôtre, bien qu'approximativement voisine. Il a localisé sur l'ADN humain le gène qui produit le "T.N.F." (Tumor Necrosis Factor). Après l'avoir cloné sur Escherichia coli et reproduit, il l'a introduit, à l'aide d'un virus vecteur, dans les lymphocytes T (Tueurs) que l'on rencontre dans les tumeurs.

Notre mécanisme thérapeutique permet de produire un clonage naturel grâce aux ARN (ribosomaux bactériens non spécifiques) opsonisés par l'adjuvant mis au point (combinaison de protéoglycanes membranaires, de collagène de type III, de chlorure de sodium).

Ce clonage induit une vaccination contre les idiotypes des anticorps anti-HIV, ainsi qu'une production d'anticorps contre le site de liaison du virus sur la molécule CD4, en particulier. Pour diminuer ou inhiber la réaction auto-immune anti CD4 (qui contribue à la lymphopénie CD4), il est nécessaire d'utiliser, lors des traitements par le complexe vaccinal, des corticoïdes (type Betaméthazone) sous forme de phosphate disodique, à la dose de 20 à 60 mg, par voie I.V. ou I.M.

Cette action s'accompagne également d'une production d'interféron endogène, ainsi que d'une activation des cellules N.K.

Le but de notre complexe vaccinal immuno-modulateur est donc d'induire une réponse immunitaire ayant pour effet d'empêcher ou au moins de réduire (jusqu'à un seuil d'auto-défense possible) la prolifération d'un agent infectieux, viral en l'occurence, introduit dans l'organisme.

Notre originalité thérapeutique consiste, entre autres, à modérer ou supprimer l'existence de "cellules suppressives" exerçant une action pro-infectieuse.

Notre traitement provoque une réaction anti-sidaïque par réponse cellulaire et/ou humorale de défense.

En conclusion, notre complexe thérapeutique agit par évolution dirigée produisant des molécules d'ARN, lesquelles, se liant à des protéines virales, bloquent l'infection par le rétrovirus du SIDA.

### Techniques d'administration du complexe vaccinal

Le complexe vaccinal peut être administré par voie orale, mais la méthode à préférer est la voie parentérale :
* soit par injection intraveineuse directe
* ou par perfusion lente
* ou encore par injection sous-cutanée.

Ces diverses techniques ont été expérimentées avec succès.

Les dosages journaliers et leur fréquence dépendent beaucoup de l'état du patient. Un surdosage ne présente aucun risque, compte tenu de la très faible toxicité du complexe.

Par voie intraveineuse, on peut utiliser des séquences d'une semaine par mois, chaque jour de la semaine de traitement comportant une perfusion lente de 500 ml d'une solution renfermant:
- 0,9 % de chlorure de sodium
- 40 µg de fractions saccharidiques membranaires (protéoglycanes de Klebsiella pneumoniae)
- 22 µg d'ARN (ribosomal) de :
   * Diplococcus pneumoniae 7µg
   * Streptococcus pyogènes (A 12) 7µg
   * Klebsiella pneumoniae 7 µg
   * Hemophilus influenzae 1 µg
- 10 µg de collagène type III décrit ci-dessus
- 8 mg de phosphate disodique de Bétaméthazone (soit 2 ml de soluté injectable)

A ce traitement par perfusion I.V. lente, peut succéder un traitement par injections sous-cutanées sur les patients pouvant être suivis de façon ambulatoire, chaque injection contenant:
- 40 µg de fractions saccharidiques membranaires (protéoglycanes de Klebsiella pneumoniae)
- 22 µg d'ARN (ribosomal) de :
   * Diplococcus pneumoniae 7µg
   * Streptococcus pyogène (A 12) 7µg
   * Klebsiella pneumoniae 7 µg
   * Hemophilus influenzae 1 µg
- 10 µg de collagène type III décrit ci-dessus
- 0,5 ml de chlorure de sodium à 0,9 %
- 4 mg de phosphate disodique de Bétaméthazone (soit 1 ml de soluté injectable).

Ce traitement peut être poursuivi plusieurs mois, jusqu'à la négativation complète de la séropositivité (c'est à dire Ag P24 = 0)

Les exemples suivants, non limitatifs, sont communiqués pour illustrer les résultats concrets de notre complexe vaccinal thérapeutique.

### Exemple 1

Mr. J. A....., 65 ans, hémophile, a été contaminé à la suite d'une transfusion reçue 8 ans auparavant. SIDA et hépatite (groupe IV C2). Une décompensation majeure se déclare en septembre 1991. Hospitalisé, il souffre d'asthénie, de perte de poids, de troubles intestinaux, rhino-sinusiens, etc... Le traitement à l'AZT se solde par un échec.

Septembre 1992 - début du traitement par notre complexe vaccinal : séquences de perfusions I.V.), suivies, en décembre 1992, par des séries d'injections sous-cutanées.

Actuellement, on constate :
- un bon état général
- une reprise de poids
- une séronégativation Ag P24.

### Exemple 2

Mme A. A....., 59 ans, épouse du patient précédent, subit une décompensation majeure en mai 1992, avec asthénie et perte de poids (groupe IV A).

Septembre 1992 - début du traitement par notre complexe vaccinal: séquences de perfusions I.V.), suivies, en décembre 1992, par des séries d'injections sous-cutanées.

Actuellement, on constate :
- un excellent état général
- la reprise du poids initial
- une séronégativation Ag P24

### Exemple 3

Mr E. C...., 30 ans, a été contaminé par voie sexuelle 6 ans auparavant. SIDA diagnostiqué en 1988 (groupe IV C1). En 1992, le patient souffre d'asthénie, puis d'un début de pneumocystose (traitement par AZT + Bactrim Forte).

Septembre 1992 - début du traitement par notre complexe vaccinal : séquences d'injections sous-cutanées uniquement, avec arrêt de toute autre thérapeutique.

Actuellement, on constate :
- un excellent état général (ayant permis une extension de l'activité professionnelle)
- une séronégativation Ag P24;

Ces traitements par le complexe vaccinal ont été pratiqués sur ces divers patients sur leur demande expresse et en raison de l'inefficacité avérée de leurs traitements en cours.

## Revendications

1. Complexe immunomodulateur antisidaïque constitué d'un mélange d'acides ribonucléïques bactériens sélectionnés, de fractions membranaires bactériennes selectionnées- glycopeptides et lipopolysaccharides- et d'acides aminés de collagéne de type III.

2. Complexe immunomodulateur selon la revendication 1, **caractérisé en ce qu'**il comprend des molécules duales constituées par le couplage d'un bras fonctionnel d'acides aminés assurant la liaison à une cible, avec un bras génétique d'ARN correspondant à la description codée de la composition du bras fonctionnel.

3. Complexe immunomodulateur selon les revendications 1 et 2, **caractérisé par** la production d'anticorps dirigés contre le site de liaison du virus, sur la molécule CD4 en particulier, et la production d'interféron endogène.

4. Complexe immunomodulateur selon les revendications 1 à 3 induisant, sur les sujets séropositifs, une vaccination anti-idiotypique contre les idiotypes des anticorps anti-HIV, ayant pour effet de négativer l'antigénémie P24.

5. Complexe immunomodulateur selon l'une des revendications 1 à 4, **caractérisé par** un conditionnement sous une forme telle qu'il peut être administré par différentes voies : perfusions, injections intraveineuses, sous-cutanées, dispositifs transdermiques ou autres, avec adjonction de chlorure de sodium.

6. Complexe immunomodulateur selon l'une des revendications 1 à 5, **caractérisé par** un conditionnement sous une forme telle qu'il permet l'administration simultanée d'anti-inflammatoires majeurs, type corticoïdes, à effets immunosuppresseurs, pour inhiber ou diminuer la réaction auto-immune anti-CD4, qui contribue à la lymphopénie CD4.

## Claims

1. Anti-AIDS immunomodulator complex constituted by a mixture of selected bacterial ribonucleic acids, selected bacterial membrane fractions - glycopeptides and lipopolysaccharides - and amino acids of type III collagen.

2. The immunomodulator complex according to claim 1, **characterized in that** it comprises dual molecules constituted by the coupling of a functional amino acid arm ensuring bonding to a target, with an RNA genetic arm corresponding to the coded description of the functional arm composition.

3. The immunomodulator complex according to claims 1 and 2, **characterized by** the production of antibodies directed against the bonding site of the virus, on the CD4 molecule in particular, and the production of endogenous interferon.

4. The immunomodulator complex according to claims 1 to 3, inducing, on seropositive patients, an anti-idiotypic vaccination against idiotypes of the anti-HIV antibodies, having for its effect negativing the antigenemy P24.

5. The immunomodulator complex according to one of claims 1 to 4, **characterized by** a dosage form such that it can be administrated by different routes: perfusions, intravenous injections, subcutaneously, transdermal devices or the like, further comprising sodium chloride.

6. The immunomodulator complex according to one of claims 1 to 5, **characterized by** a dosage form such that it can be administrated simultaneously with major antiinflammatories, cortical type, with immunosuppressive effects, to inhibit or diminish the anti-CD4 auto-immune reaction, which contributes to CD4 lymphopeny.

## Patentansprüche

1. Immunmodulationskomplex gegen Aids, der aus einer Mischung aus ausgewählten bakteriellen Ribonukleinsäuren, ausgewählten bakteriellen Membranfraktionen, -Glycopeptide und Lipopolysaccharide-, und Aminosäuren von Collagen vom Typ III besteht.

2. Immunmodulationskomplex nach Anspruch 1, **dadurch gekennzeichnet, dass** er Doppelmoleküle umfasst, die aus der Verknüpfung eines funktionellen Arms aus Aminosäuren, der die Bindung an einen Zielort sicherstellt, mit einem genetischen RNA-Arm, der der Sequenz entspricht, der für die Zusammensetzung des funktionellen Amins kodiert, bestehen.

3. Immunmodulationskomplex nach den Ansprüchen 1 und 2, **gekennzeichnet durch** die Produktion von Antikörpern, die gegen die Bindungsstelle des Virus, insbesondere auf dem CD4-Molekül, gerichtet sind und **durch** die Produktion von endogenem Interferon.

4. Immunmodulationskomplex nach den Ansprüchen 1 bis 3, der bei serumpositiven Individuen eine anti-idiotypische Vakzination gegen die Idiotypen der anti-HIV-Antikörper induziert, mit der Wirkung, dass die Antigenität P24 negativ wird.

5. Immunmodulationskomplex nach einem der Ansprüche 1 bis 4, **gekennzeichnet durch** eine Konditionierung in einer Form, in der er auf verschiedenen Wegen verabreicht werden kann: als Perfusionen, intravenöse, subcutane Injektionen, percutane Mittel oder andere, unter Zugabe von Natriumchlorid.

6. Immunmodulationskomplex nach einem der Ansprüche 1 bis 5, **gekennzeichnet durch** eine Konditionierung in einer Form, in der gleichzeitig die Verabreichung von größtenteils entzündungshemmenden Mitteln vom Typ Corticoide für die Unterdrückung von Immunantworten, um die Autoimmunreaktion von anti-CD4, die zur Lymphopenie beiträgt, zu inhibieren oder zu reduzieren, ermöglicht wird.
